# EUROPEAN PATENT APPLICATION

(11) **EP 4 563 133 A1**
(43) Date of publication of application: **04.06.2025**
(21) Application number: 23859430.3
(22) Date of filing: 30.08.2023
(51) Int. Cl.: A61G 13/08, G08B 21/04

(54) **EARLY WARNING METHOD AND SYSTEM FOR OPERATING TABLE STATE, OPERATING TABLE, AND STORAGE MEDIUM**

(30) Priority: 30.08.2022 CN 202211056687
(71) Applicant: Nanjing Mindray Bio-Medical Electronics Co., Ltd., Nanjing, Jiangsu 211111 (CN); West China Hospital, Sichuan University, Chengdu, Sichuan 610041 (CN)
(72) Inventor: LI, Chunjing, Nanjing, Jiangsu 211111 (CN); AN, Jingjing, Nanjing, Jiangsu 211111 (CN); LIU, Xinyue, Nanjing, Jiangsu 211111 (CN); QU, Weijun, Nanjing, Jiangsu 211111 (CN); WEI, Tianyi, Nanjing, Jiangsu 211111 (CN); DAI, Xiaoyu, Nanjing, Jiangsu 211111 (CN); YAO, Yunfei, Nanjing, Jiangsu 211111 (CN)
(74) Representative: KIPA AB
(86) International application number: PCT/CN2023/115969
(87) International publication number: WO 2024/046401

(57) **Abstract**

Early warning method for an operating table (501) state, which includes: acquiring operation-associated data, the operation-associated data including at least one among basic situation data of a patient, the type of the operation to be performed, and a predicted operation duration (S101); acquiring an adjusted parameter of a target module of the operating table (501), the target module being the table top or another adjustable module (S102); determining a first risk level for the operating table (501) according to the operation-associated data, the adjusted parameter, and a parameter early warning range which corresponds to the adjusted parameter, and performing notification on first information of early warning which corresponds to the first risk level (S103), where the first risk level is one of multiple preset risk levels, different risk levels correspond to different information of early warning, and different parameter early warning ranges correspond to different risk levels. The first information of early warning used for reminding of the operating table (501) is determined according to a combination of the adjusted parameter of the target module and the operation-associated data, and compared to existing solutions that only provide early warning according to a parameter value, the present solution has higher accuracy. Further provided are early warning system for an operating table (501) state, an operating table (501), and a computer-readable storage medium.

## Description

This disclosure claims priority to a Chinese patent application 202211056687.1, filed on August 30, 2022, to State Intellectual Property Office of the People's Republic of China, titled "EARLY WARNING METHOD AND SYSTEM FOR OPERATING TABLE STATE, OPERATING TABLE, AND STORAGE MEDIUM", the contents of which application are incorporated herein by reference in their entirety in this application.

### TECHNICAL FIELD

The disclosure relates to the technical field of operating table, and in particular to a method for an early warning of a state of an operating table, a system for an early warning of a state of an operating table, an operating table and a storage medium.

### BACKGROUND

With the development of surgical medical technology, remote-controlled electric operating tables have been widely used in various types of operations including laparoscopic surgeries. During the operation, according to operational requirements of a surgeon, medical staff (usually nurses) need to use a control terminal of an electric operating table (such as a remote control) to adjust adjustment parameters of one or more adjustable modules on the electric operating table, such as adjusting an inclination angle of table top (such as a certain degree to the left or a certain degree to the right), adjusting an ascending distance or a descending distance of a waist bridge (such as moving downward for a certain number of centimeters). For example, in laparoscopic surgery, in order to better expose the operating field during the laparoscopic surgery, nurses often cooperate with the surgeon to incline the operating table to a head, to feet, to left, or to right.

However, when the adjustment parameters of the adjustable modules are too large, such as the ascending distance is too high, or an inclination angle is too large, etc., varying degrees of intraoperative risks and postoperative complications would be easily caused to patients.

To address this issue, the remote controls of some electric operating tables are configured by the manufacturer to display current adjustment parameters of the adjustable modules on a screen in real time, such as an inclination angle of the table top. At the same time, early warning can be issued when the adjustment parameters of the adjustable modules exceed designated range(s), such as sounding an early warning bell, to prompt medical staff that a certain adjustment parameter is too large.

The problem with the above-mentioned prior art is that a level of risk during and after an operation is not only determined by sizes of the adjustment parameters of the adjustable modules, but is affected by a variety of related factors. The existing scheme of only an issuing warning based on the sizes of the adjustment parameters obviously cannot reflect an actual risk situation of the operating table in a current state.

### SUMMARY

Based on above-mentioned shortcoming of the prior art, this disclosure provides a method for an early warning of a state of an operating table, and an operating table, so as to provide a solution for more accurately early warning of a risk of an operating table in a designated state.

A first aspect of this disclosure provides a method for an early warning of a state of an operating table, including:
acquiring operation-associated data which corresponds to the operating table, wherein the operation-associated data includes at least one of: basic situation data of a patient, a type of an operation which is to be performed on the operating table, and an estimated duration of the operation;
acquiring an adjustment parameter of a target module of the operating table, wherein the target module is a table top of the operating table or any one adjustable module which is part of the operating table;
determining a first risk level which corresponds to the operating table according to the operation-associated data, the adjustment parameter and a parameter range of early warning, which range corresponds to the adjustment parameter; and prompting with first information of early warning;
wherein the first information of early warning corresponds to the first risk level, and the first risk level is any one of multiple preset risk levels; different risk levels correspond to different information of early warning; and different parameter ranges of early warning correspond to different risk levels.

Optionally, the method further includes:
switching the first risk level to a second risk level when a preset switching condition is satisfied, and prompting with second information of early warning;
wherein, the second information of early warning corresponds to the second risk level;
the switching condition includes at least one of: the first maintenance duration which reaches a preset duration threshold; a risk level, which corresponds to a parameter range of early warning of the adjustment parameter after adjusting the target module in the operating table, is the second risk level; current situation data is preset situation data.

A second aspect of this disclosure provides a method for an early warning of a state of an operating table, including:
acquiring a first risk level which corresponds to the operating table;
acquiring a first maintenance duration in which the operating table is maintained at the first risk level and/or current situation data of a patient;
determining first information of early warning, according to the first risk level and according to the first maintenance duration and/or the current situation data of the patient;
prompting with the first information of early warning;
wherein the first information of early warning corresponds to the first risk level, and the first risk level is any one of multiple preset risk levels; different risk levels correspond to different information of early warning; and different ranges of maintenance duration correspond to different risk levels.

Optionally, the method further includes:
switching the first risk level to a second risk level when a preset switching condition is satisfied, and prompting with second information of early warning;
wherein, the second information of early warning corresponds to the second risk level;
the switching condition includes at least one of: the first maintenance duration reaches a preset duration threshold; a risk level, which corresponds to a parameter range of early warning of the adjustment parameter after adjusting the target module in the operating table, is the second risk level; current situation data is preset situation data.

A third aspect of this disclosure provides a system for an early warning of a state of an operating table, including the operating table and a terminal;
wherein, the terminal is in communicative connection with the operating table;
the terminal is configured to execute a pre-stored computer program, so as to specifically implement the method for an early warning of a state of an operating table provided in any one of the first aspect or the second aspect of this disclosure.

Optionally, the terminal is a remote control, a wired control, a control panel of the operating table, a user terminal, a central station, a monitor, or a control of another operating table.

A fourth aspect of this disclosure provide an operating table, including a main body, and a memory and a controller which are installed at the main body;
wherein the memory is configured to store a computer program, the controller is configured to execute the computer program, so as to specifically implement the method for an early warning of a state of an operating table provided in any one of the first aspect or the second aspect of this disclosure.

A fifth aspect of this disclosure provides a computer-readable storage medium, which includes a stored computer program, wherein when the computer program is executed by a processor, a device, in which the storage medium is arranged, is controlled to execute the method for an early warning of a state of an operating table provided in any one of the first aspect or the second aspect of this disclosure.

This disclosure provides a method, system for an early warning of a state of an operating table, an operating table and a storage medium. Wherein the method includes acquiring operation-associated data which corresponds to the operating table, wherein the operation-associated data includes at least one of: basic situation data of a patient, a type of an operation which is to be performed on the operating table, and an estimated duration of the operation; acquiring an adjustment parameter of a target module of the operating table, wherein the target module is a table top of the operating table or any one adjustable module which is part of the operating table; determining a first risk level which corresponds to the operating table according to the operation-associated data, the adjustment parameter and a parameter range of early warning, which range corresponds to the adjustment parameter; and prompting with first information of early warning; wherein the first information of early warning corresponds to the first risk level, and the first risk level is any one of multiple preset risk levels; different risk levels correspond to different information of early warning; and different parameter ranges of early warning correspond to different risk levels. This solution comprehensively determines the first risk level of the operating table according to the adjustment parameter of the target module of the operating table and the operation-associated data, and then determines the corresponding first information of early warning. Compared with the existing solution that only issues a warning based on a size of a parameter, the first information of early warning of this solution can obviously better reflect a current risk situation of the operating table and has higher accuracy.

### BRIEF DESCRIPTION OF THE DRAWINGS

In order to provide a clearer explanation for embodiments of this disclosure or the technical solutions in the prior art, a brief introduction is given to the accompanying drawings required in the description of the embodiments or prior art. It is evident that the accompanying drawings in the following description are only some embodiments of this disclosure. For ordinary technical personnel in the art, other accompanying drawings can be acquired based on these drawings without any creative effort.
FIG. 1 is a flowchart of a method for an early warning of a state of an operating table provided by an embodiment of this disclosure.
FIG. 2 is an example diagram of a corresponding relationship between an adjustment parameter and a parameter range of early warning provided in an embodiment of this disclosure.
FIG. 3 is a flowchart of another method for an early warning of a state of an operating table provided by an embodiment of this disclosure.
FIG. 4 is a flowchart of a further method for an early warning of a state of an operating table provided by an embodiment of this disclosure.
FIG. 5 is a structural diagram of a system for an early warning of a state of an operating table provided in an embodiment of this disclosure.

### DESCRIPTION OF THE PREFERRED EMBODIMENTS

This disclosure is further described in detail below through specific implementation modes in conjunction with the accompanying drawings. Similar elements in different embodiments are labeled with similar element numbers. In the following embodiments, many details are described to enable this disclosure to be better understood. However, those skilled in the art can easily realize that some of the features may be omitted in different situations, or may be replaced by other elements, materials, or methods. In some cases, some operations related to this disclosure are not shown or described in the description. This is to avoid the core part of this disclosure being overwhelmed by excessive description. For those skilled in the art, it is not necessary to describe these related operations in detail. They can fully understand the related operations based on the description of this disclosure and the general technical knowledge in the field.

In addition, the features, operations or characteristics described in the description may be combined in any appropriate manner to form various embodiments. At the same time, the steps or actions in the method description may also be swapped or adjusted in order in a manner that is obvious to those skilled in the art. Therefore, the various orders in the description and the drawings are only for the purpose of clearly describing a certain embodiment and are not meant to be a necessary order unless otherwise specified that a certain order must be followed.

The serial numbers assigned to the components in this disclosure, such as "first", "second", etc., are only used to distinguish an object described and do not have any order or technical meaning. The technical terms, such as "connection" and "coupling", mentioned in this disclosure, include direct and indirect connections (couplings) unless otherwise specified.

An electric operating table, also known as an automatic operating table, is a new type of operating table that includes multiple adjustable modules. During an operation using an electric operating table, medical staff (specifically doctors or nurses) can manipulate a control terminal of the electric operating table and adjust adjustment parameter(s) of any one or more adjustable modules of the electric operating table through the control terminal, so as to enable the adjustment parameter(s) of this/these adjustable modules to satisfy a current operation requirement, thereby facilitating the doctor to perform the operation.

Exemplarily, the adjustable modules of the electric operating table may include a table top, a back board, a leg board and a waist bridge. Taking the table top as an example, adjustment parameters of the table top may include an inclination angle and a movement distance of the table top. During the operation, medical staff can adjust the table top to ascend a certain distance and incline the table top to the left for a certain angle according to a current operation requirement.

The control terminal may be a remote control, a wired control, a control panel of the operating table, a computer (PC) terminal which is in communicative connection with the operating table, a central station, a monitor, or a control of another operating table.

### Embodiment 1

During an operation on a patient, a body position of the patient is determined by a state of the operating table. If the operating table remains in a designated state for a long time, the patient is caused to maintain a fixed body position for a long time, this may cause postoperative complications of varying degrees, which is not conducive to recovery of the patient. In addition, a possibility and a severity of a postoperative complication are also related to operation-associated data, such as basic situation data of the patient, a type of the operation, and an estimated duration of the operation.

Therefore, this embodiment provides a method for an early warning of a state of an operating table, so as to prompt medical staff with preset information of early warning during an operation, according to the state of the operating table and operation-associated data.

In this embodiment and other subsequent embodiments, an operating table is used to refer to the electric operating table mentioned above. The state of the operating table may include a current adjustment parameter of an adjustable module of the operating table, such as an inclination angle of a table top.

Including this embodiment, the method described in any embodiment of this disclosure can be executed by the operating table itself (specifically, by the controller of the operating table), or by a control terminal that is communicative connection with the operating table.

For ease of understanding, unless otherwise specified, methods of this embodiment and subsequent embodiments 2 and 3 are described by taking the operating table as the executor for example.

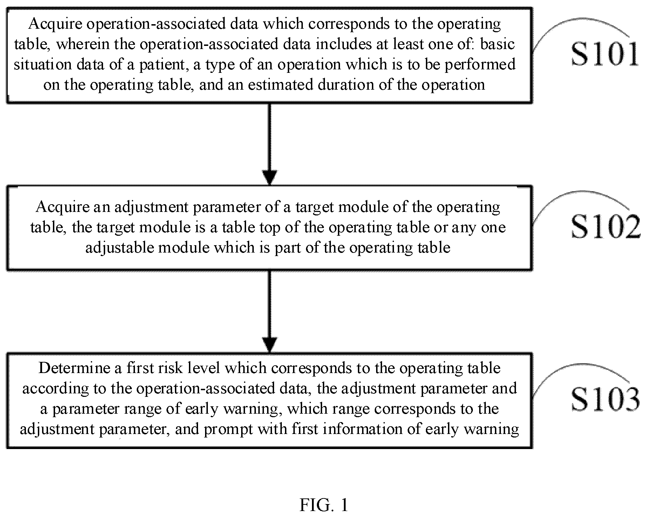

Please refer to FIG. 1, which is a flowchart of a method for an early warning of a state of an operating table provided in this embodiment. The method may specifically include the following steps.

S101, acquire operation-associated data which corresponds to the operating table, wherein the operation-associated data includes at least one of: basic situation data of a patient, a type of an operation which is to be performed on the operating table, and an estimated duration of the operation.

The basic situation data of the patient may include any one or more of: a basic physical indicator of the patient; such as gender, height, weight, age, body mass index (BMI) and cardiopulmonary function classification of the patient; underlying disease data of the patient, and previous history data of operation of the patient.

The underlying disease data of the patient is used to indicate whether the patient has or has had underlying disease(s), which include(s) but (are)is not limited to cardiovascular and cerebrovascular underlying disease, chronic pneumonia, and other common chronic diseases, as well as to indicate a severity of these(this) underlying disease(s) that the patient has or has had. For example, the underlying disease data of the patient may be that the patient suffers from severe cardiovascular and cerebrovascular underlying disease, or from mild chronic pneumonia.

Wherein, cardiovascular and cerebrovascular underlying disease specifically refers to coronary heart disease, myocardial infarction, hypertension, cerebral hemorrhage, cerebral thrombosis, cerebral embolism, etc.

The type of the operation may be any one of: general surgery, urological surgery, thoracic surgery, orthopedic surgery, cardiovascular and cerebrovascular surgery, cranial nerve surgery, gynecological and obstetric surgery, ophthalmic surgery, otolaryngology surgery and plastic surgery, or may be other types of surgery not listed here.

The estimated duration of the operation indicates how long the operation is estimated to take. The estimated duration of the operation can be set by a doctor who performs the operation before the operation begins. For example, the estimated duration of the operation may be 2 hours, which means that the doctor estimates to complete the operation in about 2 hours.

The operation-associated data which corresponds to the operating table can be acquired in a variety of ways. For example, the operating table can be connected to a data system of hospital via a wired network or a wireless network, and then the operation-associated data which corresponds to the operating table can be imported from the data system. The data system may specifically be a hospital information system (HIS), a patient monitor, a medical bracelet, or other systems without limitation.

Taking HIS system as an example, during disease diagnosis of the patient, medical staff can enter the basic situation data of the patient into the HIS system. When the patient is determined to need an operation, the doctor who performs the operation can enter the type of the operation and the estimated duration of the operation into the HIS system. Therefore, before starting the operation which uses the operating table, medical staff can start a data import function of the operating table by clicking a designated button on the control terminal of the operating table (such as "data import" button on the remote control of the operating table), and then the operating table imports the operation-associated data from the HIS system.

The operating table can also determine the operation-associated data which corresponds to the operating table, through an instruction for acquiring data. The control terminal of the operating table can generate a corresponding instruction for acquiring data according to a manipulation of the medical staff, and transmit the instruction for acquiring data to the operating table. The instruction for acquiring data may carry all or part of the operation-associated data. By receiving and parsing the instruction for acquiring data, the operating table can determine the corresponding operation-associated data.

Taking the control terminal as the control panel of the operating table as an example, medical staff can edit the operation-associated data on the control panel before the operation begins. For example, they can input weight, age, BMI, and basic situation data, such as whether having a underlying disease, of the patient, through physical buttons or virtual buttons of the control panel, and input the type of the operation and the estimated duration of the operation. The control panel generates the instruction for acquiring data, which instruction carries the above-mentioned operation-associated data, based on information inputted by the medical staff, and transmits the instruction for acquiring data to a controller of the operating table. The controller parses the above-mentioned instruction for acquiring data to acquire the operation-associated data carried therein.

When the control terminal of the operating table is used as the executor, the instruction for acquiring data can be an instruction for editing data and an instruction for entering data for the medical staff on the control terminal. The control terminal acquires the operation-associated data, which is entered by the medical staff, by parsing the instruction for acquiring data. For example, the medical staff inputs the weight of the patient as 60 kg through a key, and an instruction which is generated when the medical staff presses the key is the instruction for acquiring data. The control terminal parses the instruction for acquiring data and determines the operation-associated data that the weight of the patient is 60 kg.

In some optional embodiments, after executing step S101, whether early warning of a state is required, is determined according to the type of the operation. If a current type of operation is determined to belong to a preset type of operation that requires the early warning of a state, steps S102 and S103 are executed. If a current type of operation is determined to not belong to the preset type of operation that requires early warning of a state, steps S102 and S 103 may not be executed and this embodiment is terminated. That is to say, after acquiring the operation-associated data, only when the type of the operation is the preset type of operation, a first risk level which corresponds to the operating table is determined according to the operation-associated data, an adjustment parameter and a parameter range of early warning, which range corresponds to the adjustment parameter.

Wherein the type of the operation, which does not require the early warning of a state, can be some types of operation which have a low risk, such as an otolaryngology surgery and a plastic surgery. All types of operation, except those that do not require the early warning of a state, are types of operation which require the early warning of a state, such as the aforementioned general surgery, urological surgery, thoracic surgery, orthopedic surgery, cardiovascular and cerebrovascular surgery, cranial nerve surgery, and so on.

S102, acquire an adjustment parameter of a target module of the operating table, the target module is a table top of the operating table or any one adjustable module which is part of the operating table.

For different adjustable modules of the operating table, the adjustment parameters that need to be acquired in step S 102 are different. Moreover, since some adjustable modules of the operating table have multiple movement modes, for the same adjustable module of the operating table, the adjustment parameters that need to be acquired in step S 102 may also be different. When the target module has multiple adjustment parameters, for example, the target module has two adjustment parameters, namely, an inclination angle and a movement distance, any one or more adjustment parameters of the target module may be acquired in S102, for example the inclination angle and movement distance of the target module can be simultaneously acquired.

Exemplarily, when the target module is the table top of the operating table, the adjustment parameter of the table top may specifically be the inclination angle of the table top, or the movement distance of the table top, or the inclination angle and movement distance of the table top.

Wherein, an inclination direction of the table top can be any one of: head inclination, foot inclination, leftward inclination and rightward inclination, such that a type of the inclination angle of the table top acquired in S 102 can be any one of a head inclination angle, a foot inclination angle, a leftward inclination angle and a rightward inclination angle, of the table top. Similarly, a movement direction of the table top can be upward, downward, leftward and rightward, such that a type of the movement distance acquired in S 102 can be any one of: an upward movement distance, a downward movement distance, a leftward movement distance and rightward movement distance, of the table top. When the target module is a back board of the operating table, the adjustment parameter of the back board acquired in S 102 may specifically be a folding angle of the back board.

When the target module is a leg board of the operating table, the adjustment parameter acquired in S 102 may be a folding angle of the leg board.

When the target module is a waist bridge of the operating table, the adjustment parameter acquired in S 102 may be a movement distance of the waist bridge. A movement direction of the waist bridge can be upward or downward, and therefore, a type of the movement distance of the waist bridge can be an upward movement distance or a downward movement distance, of the waist bridge.

The above target modules are only examples. In practical applications, other adjustable modules different from the above examples may be used as target modules to acquire different adjustment parameters, which is not limited in this embodiment.

An optional way to acquire the adjustment parameter of the target module is to acquire the adjustment parameter of the target module of the operating table through detection of a sensor of the operating table.

For example, a sensor for inclination angle or a distance sensor of the operating table can be used to detect the inclination angle and the movement distance of the table top of the operating table.

An optional way to acquire the adjustment parameter of the target module is to determine the adjustment parameter of the target module of the operating table according to a control instruction for the operating table. In this way, the control instruction can directly carry the adjustment parameter of an adjusted target module, or can carry an adjustment magnitude of the adjustment parameter of the target module. For the former, the control instruction can be directly parsed to acquire the adjustment parameter of the target module after adjustment. For the latter, the adjustment parameter after adjustment can be determined according to the parsed adjustment amplitude and the adjustment parameter before receiving the control instruction.

For example, the operating table (or the control terminal of the operating table) can parse the control instruction for adjusting the table top of the operating table generated according to the manipulation of the user, so as to acquire the inclination angle and the movement distance of the table top after adjustment according to the control instruction.

Taking that the target module is the table top as an example, the medical staff manipulate the control terminal to adjust the inclination angle of the table top to 10 degrees of a head inclination angle, the operating table (or the control terminal) parses the control instruction generated thereby, so as to acquire the adjustment parameter of the table top as a head inclination angle of 10 degrees. In another example, the medical staff manipulate the control terminal to reduce the inclination angle of the table top by 5 degrees from a current head inclination angle of 15 degrees before adjustment, the operating table (or the control terminal) parses the control instruction generated thereby, so as to acquire the adjustment parameter of the table top as a head inclination angle of 10 degrees, according to the head inclination angle of 15 degrees before adjustment, and the control instruction for reducing the inclination angle of the table top by 5 degrees, that is, the head inclination angle of 15 degrees before adjustment minus the adjustment magnitude of 5 degrees.

Of course, the adjustment parameter of the target module may also be acquired in other ways different from the above two acquiring ways, and this embodiment does not limit the specific acquiring way.

In some optional embodiments, step S 102 may be performed after determining that the operating table is in a stable state. The stable state of the operating table can be understood as a state in which each adjustable module of the operating table remains stationary, that is, the adjustment parameter of the adjustable module does not change.

For example, after the operating table is started, the table top of the operating table is in a horizontal state, that is, the inclination angle is 0 degree, and then the medical staff use the control terminal of the operating table to input a control instruction for adjusting the table top of the operating table from 0 degree to 20 degrees to the left. After the operating table receives the control instruction, the table top gradually inclines to the left from the horizontal state. During this process, the leftward inclination angle of the table top gradually increases. At this time, the state of the operating table is an unstable state. When the operating table inclines to the left for 30 degrees, the table top remains still and no longer continues to incline. At this time, the state of the operating table can be regarded as a stable state.

The beneficial effect of this embodiment is that, under normal circumstances, the operation will only be performed when the operating table is in a stable state. Therefore, acquiring the adjustment parameter after determining that the operating table is in a stable state can ensure that the adjustment parameter acquired is the same as the adjustment parameter when the operation is performed, thereby preventing the risk level from being determined incorrectly due to the inconsistency between the adjustment parameter acquired and the adjustment parameter when the operation is performed.

A method for determining whether the operating table is in a stable state may be as follows. If no control instruction for adjusting the adjustable module of the operating table is acquired for a certain period of time (for example, 1 minute, or 2 minutes, which can be set as needed), then the operating table is determined to be in a stable state. Or the adjustment parameters of the various adjustable modules of the operating table are detected to determine whether they have changed, and if the adjustment parameters of the various adjustable modules of the operating table have not changed within the aforementioned certain time period, then the operating table is determined to be in a stable state.

S103, determine a first risk level which corresponds to the operating table according to the operation-associated data, the adjustment parameter and a parameter range of early warning, which range corresponds to the adjustment parameter, and prompt with first information of early warning.

First, a corresponding relationship between the adjustment parameter and the parameter range of early warning is explained.

As mentioned above, the adjustment parameter may specifically be an inclination angle, a movement distance or a folding angle. Correspondingly, when the adjustment parameter includes an inclination angle, the parameter range of early warning may include an early warning range of inclination angle; when the adjustment parameter includes a movement distance, the parameter range of early warning may include an early warning range of movement distance; when the adjustment parameter includes a folding angle, the parameter range of early warning may include an early warning range of folding angle.

Furthermore, depending on a type of the adjustment parameter, when the target modules to which the adjustment parameters belong are different, the corresponding parameter ranges of an early warning for the adjustment parameters may also be different.

Exemplarily, when the target module is the table top; early warning ranges of inclination angle, which ranges correspond to the head inclination angle, the foot inclination angle, the leftward inclination angle and the rightward inclination angle, are different, that is, different types of inclination angle correspond to different parameter ranges of early warning. An early warning range of movement distance, which rang corresponds to a movement distance when the target module is the table top, is different from an early warning range of movement distance, which rang corresponds to a movement distance when the target module is a waist bridge. An early warning range of folding angle, which range corresponds to a folding angle when the target module is a back board, is different from an early warning range of folding angle, which range corresponds to a folding angle when the target module is the leg board. That is, early warning ranges of same adjustment parameters of different modules (such as movement distances of different modules) are different. When the target module is the table top, early warning ranges of movement distance, which ranges correspond to the ascending distance, the descending distance, the left movement distance and the right movement distance, are different, that is, different types of movement distance correspond to different parameter ranges of early warning.

The first information of early warning corresponds to the first risk level, and the first risk level is any one of multiple preset risk levels. Exemplarily, the preset multiple risk levels may include three risk levels of a high risk level, a medium risk level and a low risk level. Of course, more risk levels may be set according to actual requirements, such as a ultra-high risk level, a high risk level, a secondary-high risk level and a low risk level. This application does not limit the number of risk levels.

Further optionally, the risk level can also be customized by medical staff as needed. For example, the initial operating table or control terminal is set with three risk levels: a low risk level, a medium risk level, and a high risk level. During actual use, medical staff found that the three risk levels did not match the actual operation process. Therefore, on the basis of the three risk levels of the low risk level, the medium risk level, and the high risk level, a risk level higher than the high risk is added, and named as a ultra-high risk level, and a risk level lower than the low risk is added, named as a ultra-low risk level. At this time, the first risk level can be any one of the risk levels among the ultra-low risk level, the low risk level, the medium risk level, the high risk level, and the ultra-high risk level.

Different risk levels correspond to different information of early warning. In combination with the above example, the low risk level, the medium risk level, the high risk level correspond to different information of early warning respectively. For example, information of early warning for the high risk level may be "The current risk for operation is high, please adjust operating table or end operation as soon as possible", information of early warning for the medium risk level may be "There is a certain risk for operation at present, please pay attention", and information of early warning for the low risk level may be "The current risk is low, and the operation can be performed normally". The specific content of the information of early warning can be set according to actual conditions and is not limited to the above examples.

Different parameter ranges of early warning correspond to different risk levels, or in other words, different risk levels correspond to different parameter ranges of early warning. Taking the adjustment parameter is an inclination angle as an example, an early warning range of inclination angle, which range corresponds to the low risk level, can be less than or equal to 10 degrees, an early warning range of inclination angle, which range corresponds to the medium risk level, can be greater than 10 degrees but less than or equal to 25 degrees, an early warning range of inclination angle, which range corresponds to the high risk level, can be greater than 25 degrees.

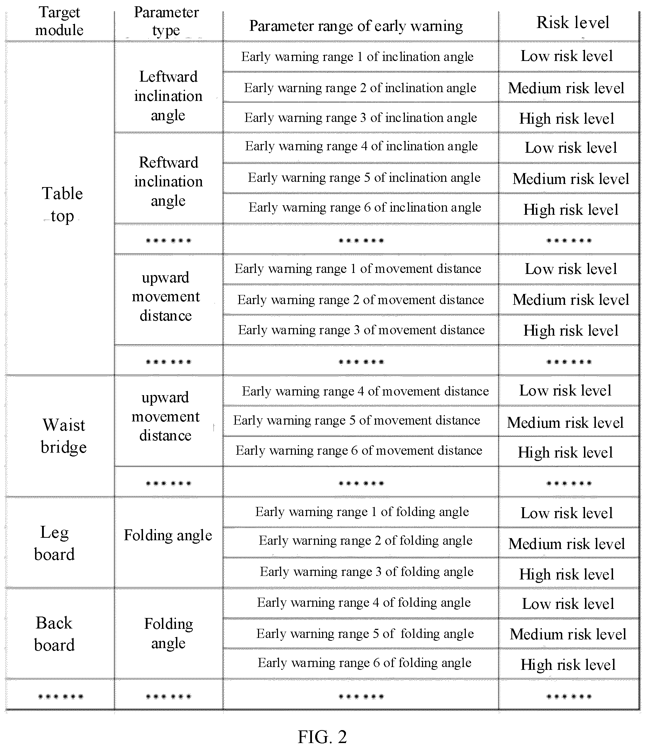

To facilitate understanding of the division of the above parameter ranges of an early warning, please refer to FIG. 2, which is an example diagram of the corresponding relationship between the adjustment parameters and the parameter ranges of an early warning in this embodiment.

As shown in FIG. 2, when the target module is a table top, and the adjustment parameter is a leftward inclination angle, the leftward inclination angle corresponds to an early warning range of inclination angle 1 to an early warning range of inclination angle 3, and the corresponding risk levels are the low risk level, the medium risk level, and the high risk level respectively. When the target module is a table top, and the adjustment parameter is a rightward inclination angle, the rightward inclination angle corresponds to an early warning range of inclination angle 4 to an early warning range of inclination angle 6, and the corresponding risk levels are the low risk level, the medium risk level, and the high risk level respectively.

When the target module is the table top, and the adjustment parameter is an ascending distance, the ascending distance corresponds to an early warning range of movement distance 1 to an early warning range of movement distance 3, and the corresponding risk levels are the low risk level, the medium risk level, and the high risk level respectively. When the target module is the table top, and the adjustment parameter is an ascending distance, the ascending distance corresponds to an early warning range of movement distance 4 to an early warning range of movement distance 6, and the corresponding risk levels are the low risk level, the medium risk level, and the high risk level respectively.

When the target module is a leg board and the adjustment parameter is a folding angle, the folding angle corresponds to an early warning range of folding angle 1 to an early warning range of folding angle 3, and the corresponding risk levels are the low risk level, the medium risk level, and the high risk level respectively. When the target module is a back board and the adjustment parameter is a folding angle, the folding angle corresponds to an early warning range of folding angle 4 to an early warning range of folding angle 6, and the corresponding risk levels are the low risk level, the medium risk level, and the high risk level respectively.

In step S103, based on the above-mentioned corresponding relationship between the adjustment parameters and the parameter ranges of an early warning, multiple parameter ranges of an early warning, which ranges correspond to different risk levels, of acquired adjustment parameters can be determined according to the types of the adjustment parameters and the target modules to which the adjustment parameters belong. Then, the first risk level can be determined, based on which parameter ranges of an early warning the adjustment parameters acquired in S102 specifically belong to, and the operation-associated data.

In the first aspect, a specific implementation method for determining a first risk level which corresponds to the operating table, according to the operation-associated data, the adjustment parameter, and a parameter range of early warning, which range corresponds to the adjustment parameter may be as follows:
acquiring a target parameter range of early prompting, within which range the adjustment parameter of the target module falls;
determining the first risk level which corresponds to the operating table, according to a risk level which corresponds to the target parameter range of early warning, and according to whether the operation-associated data satisfies a first condition.

Combined with the example of FIG. 2, assuming that the acquired adjustment parameter is the leftward inclination angle of the table top, according to the example of FIG. 2, the leftward inclination angle of the table top corresponds to the early warning range of inclination angle 1 to the early warning range of inclination angle 3. By comparing the leftward inclination angle of the table top with upper and lower limits of the early warning range of inclination angle 1 to the early warning range of inclination angle 3, a current leftward inclination angle of the table top is determined to belong to the early warning range of inclination angle 2, that is, the target parameter range of early warning, within which the leftward inclination angle of the table top falls, is the early warning range of inclination angle 2, and the corresponding risk level is the medium risk level. Therefore, the first risk level of the operating table can be determined according to the operation-associated data and the medium risk which corresponds to the early warning range of inclination angle 2.

It should be noted that the upper and lower limits of the early warning range of each parameter can be values pre-configured in a corresponding memory when the operating table or the control terminal of the operating table leaves a factory, or can be values configured by medical staff on a configuration interface of the control terminal based on experience during the use of the operating table and its control terminal. That is, the upper and lower limits of the early warning range of each parameter support customized configuration by medical staff based on experience.

In some optional embodiments, determining the first risk level which corresponds to the operating table, according to a risk level which corresponds to the target parameter range of early warning, and according to whether the operation-associated data satisfies a first condition, may include:
determining as the first risk level which corresponds to the operating table, a risk level which is higher than the risk level which corresponds to the target parameter range of early warning; when the operation-associated data satisfies the first condition;
determining as the first risk level which corresponds to the operating table, the risk level which corresponds to the target parameter range of early warning, if the risk level which corresponds to the target parameter range of early warning is a highest risk level; for example, if the risk level which corresponds to the target parameter range of early warning is already the highest risk level, the first risk level of the operating table is determined to be the highest risk level;
determining as the first risk level which corresponds to the operating table, the risk level which corresponds to the target parameter range of early warning, when the operation-associated data does not satisfy the first condition;
wherein the operation-associated data satisfying the first condition includes at least one of: the estimated duration of the operation exceeds a first duration threshold; the basic situation data exceeds preset situation data; the type of the operation is a preset type of operation.

In some other optional embodiments, determining the first risk level which corresponds to the operating table, according to a risk level which corresponds to the target parameter range of early warning, and according to whether the operation-associated data satisfies a first condition, may also include:
determining as the first risk level which corresponds to the operating table, the risk level which corresponds to the target parameter range of early warning, when the operation-associated data satisfies the first condition;
determining as the first risk level which corresponds to the operating table, a lowest risk level or a risk level which is lower than the risk level which corresponds to the target parameter range of early warning, when the operation-associated data does not satisfy the first condition.

For example, when the risk level which corresponds to the target parameter range of early warning is the high risk level; the first risk level which corresponds to the operating table is set as the high risk level, if the operation-associated data satisfies the first condition; the first risk level which corresponds to the operating table is set as the medium risk level which is lower than the high risk level or as the lowest risk level (that is, the low risk level), if the operation-associated data does not satisfy the first condition.

The risk level which corresponds to the target parameter range of early warning can be specifically determined according to the mapping relationship between the parameter range of early warning and the risk level shown in FIG. 2. For example, the leftward inclination angle of the table top is within the early warning range of inclination angle 2. According to the mapping relationship in FIG. 2, it can be determined that the risk level which corresponds to the target parameter range of early warning is the medium risk level.

The mapping relationship between the parameter range of early warning and the risk level as shown in FIG. 2 may be stored in a memory of the operating table, or in a terminal that is in communication with the operating table.

Determining as the first risk level which corresponds to the operating table, a risk level which is higher than the risk level which corresponds to the target parameter range of early warning can be that determining as the first risk level which corresponds to the operating table, a risk level which is one level higher than the risk level which corresponds to the target parameter range of early warning. For example, if the risk level which corresponds to the target parameter range of early warning is the medium risk level, determining as the first risk level which corresponds to the operating table, a risk level which is higher than the medium risk level, includes determining the high risk level as the first risk level which corresponds to the operating table. If the risk level which corresponds to the target parameter range of early warning is the low risk level, determining as the first risk level which corresponds to the operating table, a risk level which is higher than the medium risk level, includes determining the medium risk level as the first risk level which corresponds to the operating table.

Determining as the first risk level which corresponds to the operating table, a risk level which is higher than the risk level which corresponds to the target parameter range of early warning can be that directly determining the highest risk level as the first risk level which corresponds to the operating table. For example, when three risk levels of a low risk level, a medium risk level and a high risk level, are set, the high risk level can be directly determined as the first risk level of the operating table.

The first situation data may include threshold(s) of basic physical indicator(s), designated type(s) of underlying disease(s) and corresponding severity level(s) of the underlying disease(s), and a number of underlying diseases which are not said designated type(s) of underlying disease(s), etc.

The threshold(s) of basic physical indicator(s) may include a BMI threshold and an age threshold. The designated type(s) of underlying disease may be a cardiovascular and cerebrovascular underlying disease, and its corresponding severity level is severe.

When the basic situation data of the patient satisfies at least one of following four conditions: the age is greater than an age threshold, the BMI is greater than or equal to a BMI threshold, the number of other underlying disease(s) is greater than a number threshold in the first situation data, and the patient suffers from or had suffered from a designated type of underlying disease and its severity level exceeds a severity level in the first situation data or is the same as the latter, the basic situation data of the patient can be considered to exceed the first situation data.

The preset type of operation may be some types of operations that inherently have higher risks, such as cardiovascular and cerebrovascular surgery, cranial nerve surgery, etc.

A first duration threshold of the estimated duration of the operation can be set by medical staff during use, and can also be pre-entered into the operating table or the control terminal. This embodiment does not limit the specific first duration threshold. For example, the first duration threshold can be 2 hours. Optionally, multiple first duration thresholds may be set. For example, the first duration threshold may include two first duration thresholds of 1 hour and 2 hours. In this way, the first risk level may be determined according to which first duration threshold is exceeded.

It can be seen that when the operation-associated data satisfies the first condition, the operation has a higher risk level. Specifically, compared with the situation where the first condition is not satisfied, there will be a greater probability of postoperative complication, and the severity of postoperative complication is also higher. At this time, the first risk level is set as a level which is higher than the risk level which corresponds to the target parameter range of early warning, and the surgeon can take corresponding preventive measures to avoid the above risks.

In some optional embodiments, when the operation-associated data satisfies the first condition, the first risk level can be further determined according to a degree to which the basic situation data of the patient exceeds the first situation data and which of multiple first duration thresholds the estimated duration of the operation is greater than.

Exemplarily, assume that the first duration thresholds are 1 hour and 2 hours; the first situation data includes an age threshold of 60 years old, a BMI threshold of 28, having or having had severe cardiovascular and cerebrovascular underlying disease, and a number of other disease(s) being 0.

Based on the above example, in the operation-associated data, when the basic situation data of the patient includes a history of severe cardiovascular and cerebrovascular underlying disease (i.e., having suffered from severe cardiovascular and cerebrovascular underlying disease), and the estimated duration of the operation is greater than 1 hour, the basic situation data can be considered as exceeding the first situation data, and the estimated duration of the operation is greater than the first duration threshold. Therefore, the operation-associated data can be determined to satisfy the first condition, and the first risk level of the operating table is set as one level higher than the risk level which corresponds to the target parameter range of early warning.

In the operation-associated data, when the basic situation data of the patient includes a history of severe cardiovascular and cerebrovascular underlying disease (i.e., having suffered from severe cardiovascular and cerebrovascular underlying disease), and the estimated duration of the operation is greater than 2 hours, at this time, the operation-associated data satisfies the first condition, and as the estimated duration of the operation is greater than the larger of the two first duration thresholds, the first risk level is set as the highest risk level, such as the high risk level among the low risk level, the medium risk level, and the high risk level.

In the operation-associated data, when the estimated duration of the operation is greater than 2 hours, the basic situation data of the patient does not include a history of severe cardiovascular and cerebrovascular underlying disease, but satisfies at least two of the three conditions: the age is greater than an age threshold of 60 years, the BMI greater than a BMI threshold of 28, and the number of other underlying disease(s) is greater than or equal to 1; the basic situation data of the patient can also be considered as exceeding the first situation data. At this time, the first risk level can be set as one level higher than the risk level which corresponds to the target parameter range of early warning.

In the second aspect, a specific implementation method for determining a first risk level which corresponds to the operating table, according to the operation-associated data, the adjustment parameter, and a parameter range of early warning, which range corresponds to the adjustment parameter may be as follows:
acquiring at least one parameter range of early warning, which range matches with the operation-associated data and corresponds to the adjustment parameter of the target module;
determining, from the at least one parameter range of early warning, a target parameter range of early warning, within which range the adjustment parameter falls;
determining the first risk level which corresponds to the operating table, according to a risk level which corresponds to the target parameter range of early warning.

In the specific implementation method of the second aspect, the parameter range of early warning, which range corresponds to the adjustment parameter, shown in FIG. 2 can be further divided into at least one parameter range of early warning that matches different operation-associated data. After determining multiple parameter ranges of an early warning which ranges correspond to the adjustment parameter, at least one parameter range of early warning, which range matches with the operation-associated data, can be further screened out, and the parameter range of early warning, in which range the adjustment parameter falls, among these matched parameter ranges of an early warning, can be determined as the target parameter range of early warning.

Taking the early warning range of inclination angle, which range corresponds to the leftward inclination angle, in FIG. 2 as an example, the early warning range of inclination angle 1 may include an early warning range of inclination angle 1-1 that matches with operation-associated data which does not satisfy the first condition; the early warning range of inclination angle 2 may include an early warning range of inclination angle 2-1 that matches with operation-associated data which does not satisfy the first condition, and an early warning range of inclination angle 2-2 that matches with operation-associated data which satisfies the first condition. Wherein an upper limit and lower limit of the early warning range of inclination angle 2-2 may be consistent with an upper limit and lower limit of the early warning range of inclination angle 1-1. The early warning range of inclination angle 3 may include an early warning range of inclination angle 3-1 which range matches with operation-associated data which does not satisfy the first condition, and an early warning range of inclination angle 3-2 and an early warning range of inclination angle 3-3 which match with operation-associated data which satisfies the first condition. Wherein the early warning range of inclination angle 3-2 may cover the early warning range of inclination angle 2-1 and the early warning range of inclination angle 3-1, and the early warning range of inclination angle 3-3 may cover the early warning range of inclination angle 1-1 to the early warning range of inclination angle 3-1.

In combination with the example in the specific implementation method of the first aspect, the early warning range of inclination angle 1-1 can be less than or equal to 10 degrees, but greater than 5 degrees, the early warning range of inclination angle 2-1 can be greater than 10 degrees, but less than or equal to 25 degrees, and the early warning range of inclination angle 3-1 can be greater than 25 degrees.

The early warning range of inclination angle 2-2 may be less than or equal to 10 degrees, but greater than 5 degrees, and the early warning range of inclination angle 3-2 may be greater than 10 degrees. The operation-associated data, which is matched with the early warning range of inclination angle 2-2 and the early warning range of inclination angle 3-2, may include that the basic situation data of the patient includes a history of severe cardiovascular and cerebrovascular underlying disease (i.e., having suffered from severe cardiovascular and cerebrovascular underlying disease), and an estimated duration of the operation of more than 1 hour; and may include that an estimated duration of the operation of more than 2 hours, and the basic situation data of the patient satisfies at least two of following three conditions: the age is greater than the age threshold of 60 years old, the BMI is greater than the BMI threshold of 28, and the number of other underlying disease(s) is greater than or equal to 1.

The early warning range of inclination angle 3-3 covers the early warning range of inclination angle 1-1 to the early warning range of inclination angle 3-1, that is, the early warning range of inclination angle 3-3 may be greater than 5 degrees. The operation-associated data, which matches the early warning range of inclination angle 3-3, may include that the basic situation data of the patient includes a history of severe cardiovascular and cerebrovascular underlying disease (i.e., having suffered from severe cardiovascular and cerebrovascular underlying disease), and the estimated duration of the operation is greater than 2 hours. That is, when the above situation exists, as long as the table top of the operating table is inclined to the left by more than 5 degrees, the first risk level of the operating table is set as the high risk level.

Optionally, before prompting with the first information of early warning in step S103, following steps may also be performed:
acquiring a first maintenance duration in which the operating table is maintained at the first risk level, and outputting the first maintenance duration.

The first maintenance duration in which the operating table is maintained at the first risk level can be obtained through timing by a timer which is built into the operating table or the control terminal of the operating table after determining that the operating table is at the first risk level. The method for acquiring the second maintenance duration of the subsequent second risk level is similar and is not repeated here.

Optionally, prompting with the first information of early warning may include:
acquiring a first maintenance duration in which the operating table is maintained at the first risk level, and/or current situation data of the patient;
wherein the method for acquiring the first maintenance duration may refer to the above steps; the current situation data of the patient may specifically include at least one current physiological index of the patient, including but not limited to at least one of a current blood pressure, a current intracranial pressure, a current intraocular pressure, and a current bleeding volume, of the patient; wherein theses situation data may be acquired by detecting via various monitoring devices which are connected to the body of the patient during the operation;
determining first information of early warning, according to the first risk level, and according to the first maintenance duration and/or the current situation data of the patient; and
prompting with the first information of early warning.

Wherein, different maintenance durations correspond to different information of early warning. That is to say, there are multiple warning information which corresponds to the first risk level. After acquiring the first maintenance duration of the operating table at the first risk level, warning information which corresponds to the first maintenance duration can be selected from the multiple warning information which correspond to the first risk level according to the first maintenance duration, for prompting.

One way to determine different first information of early warning based on different maintenance durations may be to add the maintenance duration of the operating table at the first risk level to a content of the first information of early warning. For example, when the first risk level is the medium risk level, the first information of early warning may be "The operating table has been in a medium risk state for 3 hours, please pay attention". The "3 hours" is the maintenance duration of the operating table in the medium risk state when warning. Therefore, as the maintenance duration during warning are different, the specific warning information used for prompting will be different.

Another way to determine different first information of early warning based on different maintenance durations may be to set multiple ranges of maintenance duration for each risk level, and different ranges of maintenance duration under the same risk level correspond to different information of early warning. Thus, after acquiring the first maintenance duration of the operating table, a target range of maintenance duration for the first maintenance duration can be determined, and then information of early warning which corresponds to the first risk level and the target range of maintenance duration, can be determined as the first information of early warning.

Determining the first information of early warning according to the current situation data of the patient can be determining whether a physical health state of the patient is good or poor based on whether the current situation data of the patient satisfies preset situation data, and then adding the physical health state of the patient to the first information of early warning.

The preset situation data may include a threshold of at least one physiological indicator of the patient, such as a threshold of at least one of a blood pressure, an intracranial pressure, an intraocular pressure and a bleeding volume. If the value of the physiological indicator in the current state data of the patient exceeds the threshold in the above-mentioned preset situation data, the physical health state is determined to be poor; or else the physical health state is determined to be good.

Further optionally, when the physical health state is poor, the first information of early warning may also include indication which corresponds to the physiological indicator that exceeds the threshold. For example, if the blood pressure of the patient exceeds the blood pressure threshold in the preset situation data, the first information of early warning may include an indication that "blood pressure of patient is high."

In this embodiment, the first information of early warning can also be determined by combining the first maintenance duration and the current situation data of the patient. For example, when the first risk level is the medium risk level, the first maintenance duration reaches 3 hours, the blood pressure of the patient exceeds the threshold, and the physical health condition of the patient is poor, the first information of early warning can be "The physical condition of the patient is poor, the blood pressure is high, and the operating table has been at the medium risk level for 3 hours, please pay attention."

Optionally, outputting the first maintenance duration and prompting with the first information of early warning which corresponds to the first maintenance duration and the first risk level in step S103, can be performed regularly according to a certain timing rule after determining the first risk level of the operating table.

The timing rule can be set as needed, and this embodiment is not limited to a specific timing rule. As an example, the timing rule may be that after the operating table is in a stable state, prompt every hour in the first two hours, and prompt every half hour in the third to fourth hours, and prompt every quarter (i.e., 15 minutes) after four hours. That is to say, prompting in step S 103 may be given at the end of the 1st hour, the end of the 2rd hour, the end of the 2.5th hour, the end of the 3rd hour, the end of the 3.5th hour, the end of the 4th hour, the end of 15th minute after the fourth hour, and the end of 30th minute after the fourth hour, after the operating table is in a stable state, using the information of early warning which is determined according to the first risk level and the first maintenance duration.

The advantage of outputting the first maintenance duration and prompting with different information of early warning according to the first maintenance duration is that, by outputting the above information, medical staff can be informed of how long the operating table has been at the first risk level, thereby warning the medical staff to pay attention to the duration in which the operating table is maintained at the first risk level, avoiding intraoperative risks caused by the patient being in a fixed position for a long time, and also facilitating the medical staff to decide whether to adjust the current state of the operating table and how to adjust it based on the duration in which the operating table is maintained at the first risk level.

In some optional embodiments, before acquiring the adjustment parameter of the target module, or in other words, before the medical staff starts to adjust the target module of the operating table, it is possible to first determine whether the operation-associated data satisfies a preset third condition. If the third condition is satisfied, initial information of early warning is determined and preset, and the initial information of early warning is used for prompting. If the third condition is not satisfied, the step of acquiring the adjustment parameter of the target module is performed normally.

Optionally, the operation-associated data satisfying the third condition, means that the operation-associated data satisfies at least two of following conditions: the age of the patient is greater than an age threshold (such as 60 years old), the BMI of the patient is greater than or equal to a BMI threshold (for example, 28), the patient has a history of severe cardiovascular and cerebrovascular underlying disease, the patient has other underlying diseases, and the estimated duration of the operation is greater than 2 hours.

The initial information of early warning can be used to indicate that the current operation is risky. Please pay attention to adjusting the target module of the operating table in time. Exemplarily, the initial information of early warning may include "The patient is in poor physical condition. To avoid postoperative complications, please avoid keeping the table top at a large inclination angle for a long time."

The beneficial effect of the first embodiment is that different first risk levels and corresponding first information of early warning can be determined according to the adjustment parameter of the target module and different operation-associated data, such that the first information of early warning is capable of more realistically reflecting the risk situation during the operation.

### Embodiment 2

As the duration in which the operating table is maintained in a designated state, increases, the possibility and severity of postoperative complications in the patient generally increase accordingly. For this reason, based on the aforementioned embodiment 1, this embodiment further provides a method for an early warning of a state of an operating table, so as to intelligently adjust the risk level of the operating table and issue a corresponding early warning based on the first maintaining time in which the operating table is maintained at the first risk level.

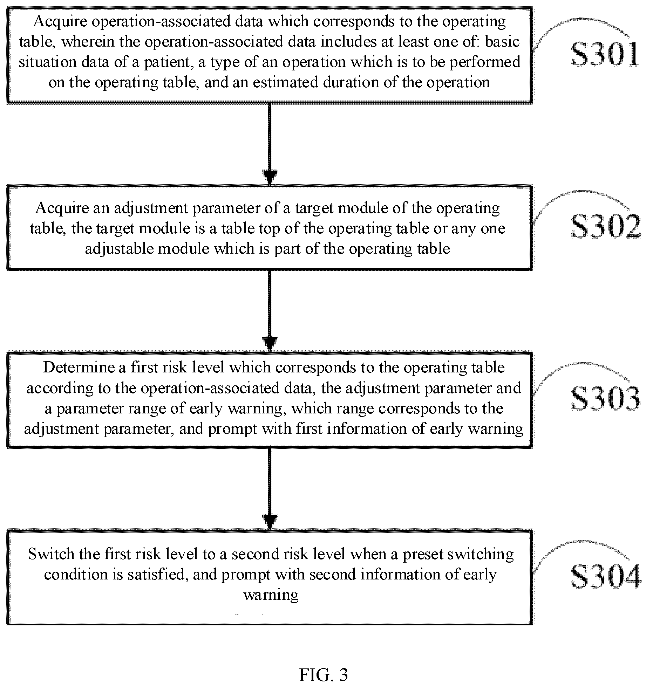

Please refer to FIG. 3 , which is another method for an early warning of a state of an operating table, including following steps.

S101, acquire operation-associated data which corresponds to the operating table, wherein the operation-associated data includes at least one of: basic situation data of a patient, a type of an operation which is to be performed on the operating table, and an estimated duration of the operation.

S302, acquire an adjustment parameter of a target module of the operating table, the target module is a table top of the operating table or any one adjustable module which is part of the operating table.

S303, determine a first risk level which corresponds to the operating table according to the operation-associated data, the adjustment parameter and a parameter range of early warning, which range corresponds to the adjustment parameter; and prompt with first information of early warning.

The specific implementation of steps S301 to S303 is consistent with steps S101 to S 103 in embodiment 1, and please refer to the above text for details, which are not repeated here.

S304, switch the first risk level to a second risk level when a preset switching condition is satisfied, and prompt with second information of early warning;
wherein, the second information of early warning corresponds to the second risk level.

It should be noted that in step S304, prompting with the second information of early warning can be carried out simultaneously with the switching of the first risk level to the second risk level, that is, prompting with the second information of early warning can be made immediately after the first risk level is switched to the second risk level; or can be carried out at different times, that is, after switching to the second risk level, prompting with the second information of early warning can be performed after a time period, such as after 1 hour.

A first switching condition for switching the risk level include at least one of: the first maintenance duration reaches a preset second duration threshold; a risk level, which corresponds to a parameter range of early warning of the adjustment parameter after adjusting the target module in the operating table, is the second risk level; the basic situation data of the patient is preset second situation data; the current situation data of the patient exceeds preset condition data. It can be understood that, in order to distinguish from the subsequent second switching condition for switching the risk level, the first switching condition for switching the risk level is used for description here, but they actually correspond to the switching condition preset in the claims.

As described above, the current situation data of the patient may include at least one of a blood pressure, an intracranial pressure, an intraocular pressure and a bleeding volume, and the preset condition data may include a threshold which corresponds to an indicator for at least one of the blood pressure, the intracranial pressure, the intraocular pressure and the bleeding volume.

Optionally, the second risk level may be one level which is higher than the first risk level, or may be one level which is lower than the first risk level, or may be the highest level among multiple preset risk levels, or may be the lowest level among multiple preset risk levels.

In a first optional embodiment, satisfying the first switching condition for switching the risk level can be that, after the target module is adjusted, a risk level, which corresponds to a parameter range of early warning, within which the adjustment parameter of the target module falls, is the second risk level.

After determining that the operating table is at the first risk level, medical staff can adjust the target module of the operating table according to an actual condition during the operation, thereby changing a value of the adjustment parameter of the target module. After each adjustment, the adjustment parameter of the adjusted target module can be detected based on the method of acquiring the adjustment parameter in step S102. If, after a certain adjustment, it is found that the risk level, which corresponds to the parameter range of early warning to which the adjusted adjustment parameter belongs, has become the second risk level, the operating table is determined to satisfy the first switching condition for switching the risk level and the operating table is switched from the current first risk level to the second risk level.

Adjustment of the target module generally needs to be implemented through control instruction(s) issued by the control terminal of the operating table. Therefore, the control instruction for adjusting the target module can be generated at the control terminal. Optionally, after receiving the control instruction for adjusting the target module, which instruction is transmitted by the control terminal, the operating table determines to start adjusting the target module. Then the adjustment is determined as being completed after the control instruction is generated or received for a certain time (for example, 5 minutes). Then the adjusted adjustment parameter of the target module is acquired in the manner described in step S102.

Take FIG. 2 as an example. Assuming that after the operating table is started, by executing steps S301 to S303, a leftward inclination angle of the table top is determined as falling within the early warning range of inclination angle 1 of FIG. 2, and then the operating table is determined as currently being in a low-risk state (that is, the first risk level is the low risk level). After the operation has been performed for one hour, a body position of the patient needs to be changed for a next stage of the operation, such that the medical staff adjusts the inclination angle of the table top through the control terminal. After the adjustment, the leftward inclination angle of the table top falls within the early warning range of inclination angle 2 of FIG. 2, such that the operating table is determined as satisfying the first switching condition for switching the risk level, and the operating table is switched from the first risk level to the second risk level which corresponds to the early warning range of inclination angle 2, that is, the operating table is switched from the low risk level to the medium risk level.

In a second optional embodiment, satisfying the first switching condition for switching the risk level, may be that the first maintenance duration reaches a second duration threshold, and the basic situation data of the patient is second situation data.

The second state data may be the same as or different from the first state data of the first embodiment. Similar to the first situation data, the second situation data may include threshold(s) of basic physical indicator(s) (such as an age threshold, a BMI threshold), designated type(s) of underlying disease(s) and corresponding severity level, a number of underlying diseases which are not said designated type(s) of underlying disease(s), etc.

Exemplarily, the second duration threshold may be 1 hour or 2 hours; the second situation data includes an age threshold of 60 years old, a BMI threshold of 28, having or having had severe cardiovascular and cerebrovascular underlying disease, and a number of other disease(s) being 0.

Based on the above example, when the basic situation data of the patient includes a history of severe cardiovascular and cerebrovascular underlying disease (i.e., having suffered from severe cardiovascular and cerebrovascular underlying disease), the basic situation data of the patient can be determined as the second situation data. At this time, if the first maintenance duration is further determined to reach the second duration threshold of 1 hour, the first switching condition for switching the risk level is determined to be satisfied, and the operating table is switched from the original first risk level to the second risk level. In this case, the second risk level may be one level higher than the first risk level. For example, if the first risk level is the low risk level, the second risk level may be the medium risk level.

When the basic situation data of the patient includes a history of severe cardiovascular and cerebrovascular underlying disease (i.e., having suffered from severe cardiovascular and cerebrovascular underlying disease), the basic situation data of the patient can be determined to be the second situation data. At this time, if the first maintenance duration is further determined to reach the second duration threshold of 2 hours, the first switching condition for switching the risk level is determined to be satisfied, and the operating table is switched from the first risk level to the second risk level. In this case, the second risk level may be the highest level among the preset multiple risk levels, for example, it may be the high risk level among the low risk level, the medium risk level and the high risk level.

When the basic situation data of the patient satisfies at least two of three conditions: the age is greater than an age threshold of 60 years, the BMI is greater than a BMI threshold of 28, and the number of other underlying disease(s) is greater than or equal to 1, the basic situation data of the patient can be considered as the second situation data. At this time, if the first maintenance duration is determined to reach the second duration threshold of 2 hours, the first switching condition for switching the risk level is determined to be satisfied, and the operating table is switched from the first risk level to the second risk level. In this case, the second risk level may be one level higher than the first risk level. For example, if the first risk level is the medium risk level, the second risk level may be the high risk level.

The second duration threshold in the first switching condition for switching the risk level may be related to the operation-associated data which corresponds to the operating table, that is, the second duration threshold may be determined according to the operation-associated data. The operation-associated data includes at least one of: basic situation data of the patient, a type of the operation which is to be performed on the operating table, and an estimated duration of the operation.

For example, if the type of the operation to be performed is a higher risk type, such as cardiovascular surgery or cranial nerve surgery, a shorter second duration threshold can be set, for example the second duration threshold can be set to 1 hour or 1.5 hours. If the type of the operation to be performed is a lower risk type, such as general surgery or orthopedic surgery, a longer second duration threshold can be set, for example the second duration threshold can be set to 2 hours.

When the estimated duration of the operation is longer, a longer second duration threshold may be set accordingly; and when the estimated duration of the operation is shorter, a shorter second duration threshold may be set accordingly.

When the basic situation data of the patient satisfies at least two of following conditions: the age is greater than a certain age threshold (such as 60 years old), the BMI is greater than a certain BMI threshold (such as 28), and the patient suffers from another underlying disease besides cardiovascular and cerebrovascular underlying disease; or when the basic situation data of the patient satisfies conditions that the patient has a history of severe cardiovascular and cerebrovascular underlying disease; a shorter second duration threshold can be set. When the basic situation data of the patient does not satisfy the above condition, a longer second duration threshold can be set.

The following describes an optional method for determining information of early warning in any embodiment of this disclosure.

In any embodiment of this disclosure, the information of early warning for prompting (including but not limited to the aforementioned first information of early warning and the second information of early warning) may include one or more of: a current risk level, a maintenance duration of the operating table at the current risk level, a physical health state which is determined according to the current situation data of the patient, or a physiological indicator in the current situation data of the patient which indicator exceeds a threshold of preset condition data, a dangerous consequence of the current risk level, and a suggestion for an intervention measure which corresponds to the current risk level; wherein the suggestion for an intervention measure includes a suggestion for adjusting the operating table and/or a suggestion for accelerating the operation.

In actual applications, the specific items mentioned above and specific contents of each item can be determined, based on the risk level of the operating table and the maintenance duration of the operating table at the current risk level when warning. The corresponding contents are then combined together to form the information of early warning which corresponds to the current risk level.

In some optional embodiments, if the risk level of the operating table is the low risk lever when warning, the information of early warning when warning, may only include the current risk level and the maintenance duration of the operating table at the current risk level; if the risk level of the operating table is the medium risk level when warning, the information of early warning when warning may further include the dangerous consequence of the current risk level on the basis of the first two items; if the risk level of the operating table is the high risk level when warning, the information of early warning when warning may further include a suggestion for an intervention measure on the basis of the first three items.

In other optional embodiments, if the risk level of the operating table is the low risk level and a duration of the low risk level is greater than a third duration threshold, or the risk level is the medium risk level; then the information of early warning when warning may include the current risk level, the duration of the operating table at the current risk level, and the dangerous consequence of the current risk level. If the risk level of the operating table is the medium risk level and the duration of medium risk level is greater than the third duration threshold, or the risk level is the high risk level; then the information of early warning when warning may include the current risk level, the duration of the operating table at the current risk level, the dangerous consequence of the current risk level, and the suggestion for an intervention measure which corresponds to the current risk level.

It should be noted that the above-mentioned items in the information of early warning can be determined separately. For example, after switching from the first risk level to the second risk level, when determining the second information of early warning, the current risk level and the maintenance duration of the operating table at the current risk level in the information of early warning can be determined according to the second risk level and the corresponding second maintenance duration. Then, the content of the suggestion for an intervention measure which corresponds to the current risk level can be determined according to the first risk level and its first maintenance duration, as well as according to the second risk level and its second maintenance duration.

The dangerous consequence, and the suggestion for an intervention measure for the current risk level, can be determined according to a preset corresponding relationship. Specifically, at least one risk level may be preset to correspond to a designated dangerous consequence, and at least one suggestion for an intervention measure may be preset to correspond to the risk level. For example, the dangerous consequence, which corresponds to the medium risk level, includes "may cause surgical complication", and the corresponding intervention measure includes " recommend to reduce the risk level". The dangerous consequence, which corresponds to the high risk level, includes "high intraoperative risk level and possible serious postoperative complication", and the corresponding intervention measure includes "recommend to appropriately reduce the inclination angle of the operating table", "please end the operation as soon as possible" and "recommend to reduce the risk level".

In this way, when it is necessary to determine the current risk level (which may be the first risk level or the second risk level mentioned above), the dangerous consequence and the intervention measure which correspond to the current risk level can be found according to the above corresponding relationship, and this information can be combined with the current risk level and its maintenance duration into corresponding information of early warning.

It is understandable that if the current risk level has no corresponding dangerous consequence and intervention measure, such as there are no dangerous consequence and intervention measure which correspond to the low risk level in the above example, then it is determined that the information of early warning which corresponds to the current risk level does not include these two items. When a risk level corresponds to multiple dangerous consequences or multiple intervention measures, any one of them can be selected to be added to the information of early warning. For example, in the above example, the intervention measures which correspond to the high risk level include "Recommend to appropriately reduce the inclination angle of the table", "Please end the operation as soon as possible" and "Recommend to reduce the risk level". At this time, any one of them can be selected as the suggestion for an intervention measure in the information of early warning during warning.

Further optionally, the dangerous consequence and the suggestion for an intervention measure which correspond to the risk level can also be set with a corresponding applicable condition, and they will only be added to the information of early warning when the applicable condition is satisfied. For example, the suggestion for an intervention measure which corresponds to the above-mentioned medium risk level is "Recommend to lower the risk level", and its applicable condition can be set as the maintenance duration of the medium risk level is greater than 2 hours. That is to say, only when the operating table is maintained at the medium risk level for more than 2 hours, the information of early warning which corresponds to the medium risk level used for promoting includes the suggestion for an intervention measure of "Recommend to lower the risk level". Before this, the information of early warning of the medium risk level does not include this suggestion for an intervention measure.

The third duration threshold may be a threshold preset in a memory of the operating table or a memory of the control terminal of the operating table when the operating table leaves the factory, or may be a threshold configured by the medical staff through the control terminal during the use of the operating table.

Exemplarily, the third duration threshold may be 2 hours.

Here are some examples of content of the information of early warning.

Example 1: The operating table is at the low risk level, and the maintenance duration of the operating table at the low risk level is 1 hour. The information of early warning may include "The operating table has been at a low risk level for the past hour and the operation can be performed normally", wherein "low risk level" and "1 hour" are the current risk level of the operating table and the maintenance duration of the current risk level.

Example 2: The operating table is at a low-risk level, and the maintenance duration of the operating table at the low risk level is 3 hours, which is greater than the third duration threshold. The information of early warning may include "The operating table has been at low risk level for 3 consecutive hours, which may lead to postoperative complication, please pay attention", among which "may lead to postoperative complication" is a dangerous consequence of the current risk level.

Example 3: The operating table is at a medium risk level, and the maintenance duration of the operating table at the medium risk level is 1 hour. The information of early warning may include "The operating table has been at a medium risk level for 1 hour, which may lead to postoperative complication, please pay attention."

Example 4: The operating table is at a high risk level, and the maintenance duration of the operating table at the high risk level is 1 hour, then the information of early warning may include "The operating table has been at high risk level for 1 hour, the intraoperative risk level is high and there may be serious postoperative complications. Recommend to reduce the inclination angle of the table top appropriately"; or may include "The operating table has been at high risk level for 1 hour, the intraoperative risk level is high and there may be serious postoperative complications. Please end the operation as soon as possible"; or may include "The operating table has been at high risk level for 1 hour, the intraoperative risk level is high and there may be serious postoperative complications. Recommend to lower the risk level", among which "the intraoperative risk level is high and there may be serious postoperative complications" belongs to the dangerous consequence of the current risk level, and "Recommend to reduce the inclination angle of the table top appropriately", "Please end the operation as soon as possible" and "Recommend to lower the risk level" are suggestions for an intervention measure.

Example 5: The operating table is at a medium risk level, and the maintenance duration of the operating table at the medium risk level is 2 hours. The information of early warning may include "The operating table has been at medium risk level for 2 hours. The intraoperative risk level is high and there may be serious postoperative complications. Recommend to lower the risk level."

Example 6: The operating table is at a medium risk level, and the maintenance duration of the operating table at the medium risk level is 1 hour. Before the medium risk level, the operating table was also maintained at a high risk level for 1 hour. The information of early warning determined at this time may be "The operating table is at the medium risk level for another hour after being at the high risk level. The intraoperative risk level is high and there may be serious postoperative complications. Recommend to adjust the risk level to the lowest level."

Example 7: The operating table is at a medium risk level, and the maintenance duration of the operating table at the medium risk level is 2 hours. In the current state data of the patient, the intracranial pressure exceeds the intracranial pressure threshold which is specified by the preset situation data. The information of early warning may include "The intracranial pressure of the patient is high, the operating table has been at medium risk level for 2 hours, the intraoperative risk level is high and there may be serious postoperative complications. Recommend to lower the risk level."

In addition to the above content, the information of early warning of this embodiment may also include other content, such as the adjustment parameter of the target module of the operating table when warning. Taking the above-mentioned example 4 as an example, if the operating table is at a high risk level and the maintenance duration of the high risk level is 2 hours, the information of early warning may include "The current inclination angle of the table top is 26 degrees, the operating table has been at the high risk level for 2 hours, the intraoperative risk level is high and there may be serious postoperative complications. Recommend to reduce the inclination angle of the table top appropriately", wherein " The current inclination angle of the table top is 26 degrees" is the current adjustment parameter of the target module table.

Further, referring to the aforementioned step S304, when switching the risk level, that is, switching from the first risk level to the second risk level, second information of early warning which corresponds to the second risk level after the switching can be determined only according to the second risk level after the switching, or can be determined according to the first risk level before the switching and the second risk level after the switching. The second information of early warning can also be determined according to the second risk level, as well as according to the current situation data of the patient and/or the second maintenance duration of the operating table at the second risk level.

In the first situation, the second information of early warning is determined according to the second risk level and the second maintenance duration of the operating table at the second risk level, and the second information of early warning is used for promoting.

Similar to the method of determining the first information of early warning based on the first risk level and its first maintenance duration in Embodiment 1, in the first situation, the second information of early warning may include the second risk level of the current operating table and the second maintenance duration at the second risk level. In this way, when determining the second information of early warning, contents of the second information of early warning are different depending on different second maintenance durations. For example, assuming that the second risk level is the high risk level, if the second maintenance duration is 1.5 hours, the determined second information of early warning may be "The state of the operating table has been maintained at the high risk level for 90 minutes, please complete the operation as soon as possible"; if the second maintenance duration is 3 hours, the determined second information of early warning may be " The state of the operating table has been maintained at the high risk level for 3 hours, please complete the operation as soon as possible".

In the second situation, the second information of early warning is determined according to the first risk level and the first maintenance duration, as well as according to the second risk level, the second maintenance duration in which the operating table is maintained at the second risk level, and the current situation data of the patient; and the second information of early warning is used for promoting.

In the second situation, the former method of determining the second information of early warning can be as follows: determining the second information of early warning which includes the first risk level and its first maintenance duration, the second risk level and its second maintenance duration, and a physical health state of the patient, or at least one physiological indicator in the current situation data of the patient.

Wherein, the physical health state of the patient can be determined according to whether the current situation data of the patient exceeds the preset condition data. Please refer to the previous text for the specific determination method.

For example, assuming that the first risk level is the medium risk level and the first maintenance duration is 0.5 hour, the second risk level is the high risk level and the second maintenance duration is 1.5 hours, the second information of early warning determined can be "The patient is in a poor health state, the operating table has been in the medium risk level for half an hour and had been in the high risk level for 90 minutes. Please complete the operation as soon as possible", or can be "The current intracranial pressure of the patient is high, the operating table has been in the medium risk level for half an hour and had been in the high risk level for 90 minutes. Please complete the operation as soon as possible."

In the second situation, the latter method of determining the second information of early warning may be as follows: first determining a comprehensive maintenance duration based on the first maintenance duration and the second maintenance duration, and then determining the second information of early warning according to the second risk level and the comprehensive maintenance duration, and adding the physical health state of the patient or at least one physiological indicator in the current situation data of the patient to the second information of early warning.

Optionally, the comprehensive maintenance duration may be equal to a sum of the first maintenance duration and the second maintenance duration, or the comprehensive maintenance duration may be equal to a sum of a product of the first maintenance duration and a specific weight, and the second maintenance duration.

As a first example of the latter method of determining the second information of early warning, assuming that the first risk level is the medium risk level, the first maintenance duration is 0.5 hour, the second risk level is the high risk level, and the second maintenance duration is 1.5 hours, the determined second information of early warning can be "The intracranial pressure of the patient is high, and the operating table has been at a high risk level for 2 consecutive hours. Please complete the operation as soon as possible." The 2 hours is the comprehensive maintenance duration acquired by adding the first maintenance duration of 0.5 hour and the second maintenance duration of 1.5 hours.

As a second example of the latter method of determining the second information of early warning, assuming that the first risk level is the medium risk level, the first maintenance duration is 1 hour, the second risk level is the high risk level, and the second maintenance duration is 1 hour, the determined second information of early warning can be "The intracranial pressure of the patient is high, and the operating table has been at a high risk level state for 90 consecutive minutes. Please complete the operation as soon as possible." The 90 minutes is the comprehensive maintenance duration, which is obtained by adding 30 minutes, which is obtained by the first maintenance duration of 1 hour multiplied by a weight of 0.5, with the second maintenance duration of 1 hour.

Optionally, the second information of early warning, which is determined according to the second risk level and the comprehensive maintenance duration, may be the same as the second information of early warning, which is determined according to the second risk level and the second maintenance duration which is equal to the comprehensive maintenance duration.

For example, when the second risk level is the high risk level and the second maintenance duration is 2 hours, the corresponding second information of early warning may be "The physical condition of the patient is poor, the operating table has been at a high risk level state for 2 consecutive hours, and there is a high risk level of postoperative complications. Please complete the operation as soon as possible."

When the first risk level is the medium risk level and the first maintenance duration is 0.5 hour, and the second risk level is the high risk level and the second maintenance duration is 1.5 hours, combined with the previous text, it can be determined that the comprehensive maintenance duration is 2 hours. At this time, the second information of early warning, which is determined according to the second risk level and the comprehensive maintenance duration, can be consistent with the previous warning information, which is determined according to the second maintenance duration of 2 hours, that is, "The physical condition of the patient is poor, the operating table has been at a high risk level state for 2 consecutive hours, and there is a high risk level of postoperative complications. Please complete the operation as soon as possible."

In the third situation, the second information of early warning is determined according to the second risk level, as well as according to the current situation data of the patient and/or the second maintenance duration of the operating table at the second risk level; and the second information of early warning is used for promoting.

In the third situation, the second risk level and the physical health state, which is determined according to the current situation data of the patient, may constitute the second information of early warning; or the second risk level and the physiological indicator in the current situation data of the patient, which indicator exceeds the threshold of the preset condition data, may constitute the second information of early warning; or the second risk level and the second maintenance duration of the second risk level may constitute the second information of early warning; or the second risk level, the second maintenance duration, and the physical health state, or the physiological indicator that exceeds the threshold, may also constitute the second information of early warning.

In actual applications, which of the above situations is applicable to the second information of early warning, can be determined according to a relationship between the first risk level and the second risk level, as well as the current situation data of the patient. If the second risk level is higher than the first risk level, for example, the second risk level is the high risk level and the first risk level is the medium risk level, the second information of early warning is determined according to the description of the second situation, that is, the first maintenance duration and the second maintenance duration are comprehensively considered. If the second risk level is lower than the first risk level, for example, the second risk level is the medium risk level and the first risk level is the high risk level, the second information of early warning is determined according to the description of the first situation or the third situation. Furthermore, if the physical health state of the patient is determined to be good according to the current situation data of the patient, the second information of early warning is determined according to the first situation, that is, only the second maintenance duration is considered. If the physical health condition of the patient is determined to be poor according to the current situation data of the patient, the second information of early warning is determined according to the third situation, that is, according to the second risk level, and according to the current situation data of the patient and/or the second maintenance duration of operating table at the second risk level.

In addition to the above-mentioned method of combining several items which correspond to the current risk level, it is also possible to preset multiple corresponding warning information for each risk level, and then set applicable conditions for the multiple warning information which corresponds to the same risk level. After determining the current risk level of the operating table, the information of early warning, that satisfies the applicable condition among the multiple warning information which corresponds to the risk level, can be found and used as the information of early warning for promoting.

The above applicable conditions may include at least one of the following.

Whether a maintenance duration of the operating table at a current risk level is within a designated range of the maintenance duration, whether a risk level has been switched during this operation, whether the risk level before the switching is higher or lower than the current risk level, whether the maintenance duration of the risk level before the switching is within the designated range of the maintenance duration, and whether a sum of a maintenance duration of the risk level before the switching and the maintenance duration of the current risk level is within a designated range of the maintenance duration.

The following is an explanation of how to use information of early warning to prompt.

It should be noted that when prompting with designated warning information, a variety of warning methods can be used, including but not limited to one or more of indication voice, indication text, indication icon, action of indication light and playing alarm sound.

The indication voice and indication text may specifically include the content in the aforementioned example of the information of early warning. It should be noted that the above-mentioned indication voice and indication text can be output through any one or more electronic devices, such as through the operating table, the control terminal of the operating table, and a third-party terminal device that is in communicative connection with the operating table and is not the control terminal of the operating table (for example, a smart phone of a medical staff connected to the operating table via Bluetooth).

The action of indication light may be to light up indication lights of different colors (specifically, LED lights or other indication lights) according to the risk level of the operating table when warning. For example, if the current risk level of the operating table is the low risk level when warning, a blue indication light will be turned on; if the risk level of the operating table is the medium risk level when warning, a yellow indication light will be turned on; if the risk level of the operating table is the high risk level when warning, a red indication light will be turned on.

The alarm sound can be played according to different patterns based on the risk level of the operating table when warning. For example, if the risk level of the operating table is the low risk level when warning, the alarm will ring once every 2 seconds, each ringing will last for 2 seconds, and the alarm will ring three times; if the risk level of the operating table is the medium risk level when warning, the alarm will ring once every 2 seconds, each ringing will last for 4 seconds, and the alarm will ring three times; if the risk level of the operating table is the high risk level when warning, the alarm will continue to ring for 6 seconds and then stop.

The indication icon can be displayed on a terminal that is in communicative connection with the operating table. Specifically, different indication icons which correspond to different risk levels can be pre-set. After determining the first risk level or the second risk level of the operating table, the indication icon of the first risk level can be displayed on the display screen of the terminal, or the indication icon which corresponds to the second risk level can be displayed on the display screen of the terminal.

In some optional embodiments, the alarm sound may also be a prerecorded music clip, and different risk levels correspond to different music clips when warning.

In addition to the above methods, warnings can also be given by means of: vibration of the control terminal of the operating table (such as vibration of the remote control) or vibration of a third-party terminal device (such as vibration of a mobile phone of the medical staff); or emission of a screen backlight of a designated color (such as blue backlight for the low risk level, yellow backlight for the medium risk level, and red backlight for the high risk level) by the third-party terminal device or the control terminal of the operating table; or display of images of different colors (such as a blue diagram of the operating table for the low risk level, a yellow diagram of the operating table for the medium risk level, and a red diagram of the operating table for the high risk level) by the third-party terminal device or the control terminal of the operating table, etc. This embodiment does not limit the specific warning method.

Similar to step S103, in step S304, the second information of early warning is used for promoting, or after the risk level of the operating table is switched to the second risk level, multiple warnings are made according to a certain timing rule.

Optionally, after the risk level is switched, referring to Embodiment 1, warnings can still be given regularly according to a certain timing rule, and there can be two warning schemes. The former scheme can continue timing from the first risk level as before, and the latter scheme can restart timing from when the first risk level is switched to the second risk level.

For the former scheme, combined with the setting of the timing rule in the aforementioned embodiment 1, after the first risk level is switched to the second risk level and the operating table is in a stable state, the timing is still based on the regulation of warning once every hour in the first two hours, warning once every half hour in the third to fourth hours, and warning once every quarter (i.e. 15 minutes) after four hours. For example, assuming that the operating table is switched from the first risk level to the second risk level between the 2nd hour and 2.5th hour, warnings are given based on the information of early warning which corresponds to the second risk level at the end of the 2.5th hour, the end of the 3rd hour, the end of 3.5th hours, the end of the 4th hour, the end of 15th minute after the fourth hour, and the end of 30th minute after the fourth hour, starting from when the operating table is in a stable state.

For the latter option, assuming that the operating table is switched from the first risk level to the second risk level between the 2nd hour and the 2.5th hour, then after timing from the switching for one hour, a warning is given according to the information of early warning which corresponds to the second risk level. Then after timing from the switching for two hours, another warning is given according to the information of early warning which corresponds to the second risk level, and after timing from the switching for 2.5 hours, another warning is given according to the information of early warning which corresponds to the second risk level. That is, the starting point of the timing is changed from when the operating table is in a stable state before the switching to when the switching from the first risk level to the second risk level is made.

Further optionally, the preset timing rule may be adjusted according to the first risk level and the second risk level.

For example, after switching from the first risk level to the second risk level, the preset timing rule is that, starting from when switching to the second risk level, the warning is given every 1 hour in the first two hours, and every half hour after two hours. However, when the first risk level is the medium risk level and the first maintenance duration of the first risk level is 0.5 hour, and the second risk level is the high risk level, the timing rule can be adjusted as: starting from when switching to the second risk level, a warning is given at the end of 0.5 hour and the end of 1.5 hours respectively, and then every 0.5 hour after 1.5 hours.

Although the example of the operating table for switching from the first risk level to the second risk level is used for explanation, in the actual application scenario of this embodiment, after switching to the second risk level, it is also possible to switch from the second risk level to a third risk level when a second switching condition for switching the risk level is satisfied, and switch from the third risk level to the fourth risk level when a third switching condition for switching the risk level is satisfied, and so on. The number of switching between risk levels during the operation in this embodiment is not limited.

The switching condition for each level may be the same as or different from the condition for the previous switching. For example, the second switching condition for switching the risk level may be the same as or different from the first switching condition for switching the risk level. For example, the second switching condition for switching the risk level may include that after the target module of the operating table is adjusted, the risk level which corresponds to the parameter range of early warning of the adjustment parameter is the third risk level.

After each switching of risk level, the information of early warning which corresponds to the new risk level may be determined by referring to the method for determining the second information of early warning in this embodiment. For example, after switching from the second risk level to the third risk level, third information of early warning which corresponds to the third risk level can be determined according to the third risk level and a third maintenance duration of the operating table at the third risk level. The third information of early warning can also be determined according to the third risk level and its third maintenance duration, as well as according to the second risk level and its second maintenance duration.

In some optional embodiments, when the operating table is connected with an operation robot, the information of early warning determined in any embodiment can be transmitted directly from the operating table or the control terminal of the operating table to the operation robot, thereby triggering the operation robot to adjust the adjustment parameter of the target module of the operating table according to a preset program.

The beneficial effect of the second embodiment is that it can automatically switch the risk level of the operating table according to the maintenance duration of the first risk level, the adjustment parameter of the target module and other information, and prompt according to the second information of early warning which corresponds to the second risk level after the switching, which helps to assist medical staff to understand the changes of the risk level during the ongoing operation, so as to take corresponding measures in time.

### Embodiment 3

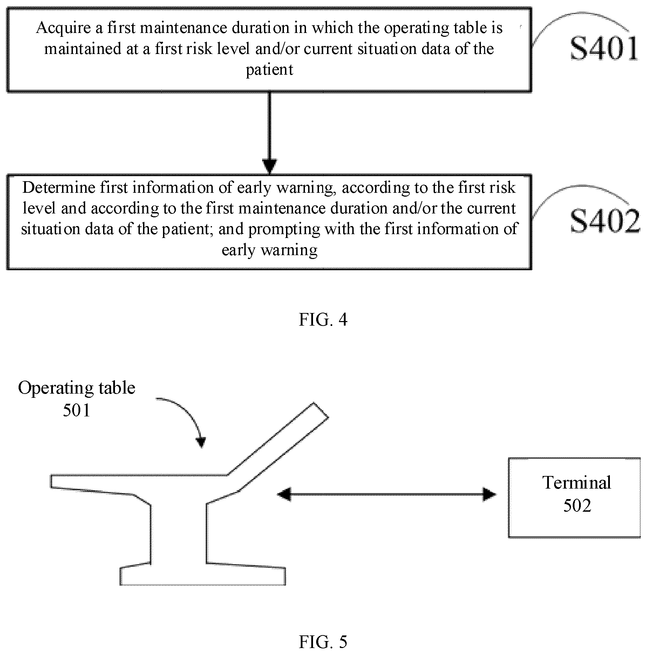

Please refer to FIG. 4, a flowchart of a further method for an early warning of a state of an operating table provided by an embodiment of this disclosure. The method may include the following steps.

S401, acquire a first maintenance duration in which the operating table is maintained at a first risk level and/or current situation data of the patient. For the specific implementation method of acquiring the first risk level which corresponds to the operating table in step S401, please refer to steps S101 to S103 in Example 1, or adopt the existing scheme for determining the risk level according to the adjustment parameter of the target module of the operating table; or adopt other schemes for determining the risk level in the operating table. The method of acquiring the first maintenance duration and the current state data of the patient can also refer to the step of acquiring the first maintenance duration of the operating table at the first risk level and the current state data of the patient in Embodiment 1, which are not repeated here.

S402, determine first information of early warning, according to the first risk level and according to the first maintenance duration and/or the current situation data of the patient; and prompting with the first information of early warning.

Wherein, the first information of early warning corresponds to the first risk level; the first risk level is any one of preset multiple risk levels; different ranges of maintenance duration correspond to different information of early warning; different risk levels correspond to different information of early warning.

The specific implementation of step S402 can refer to the method of determining the first information of early warning and/or the second information of early warning in Embodiment 1 and Embodiment 2, which are not described in detail.

In this embodiment, after executing step S402, the first risk level can be switched to the second risk level when the operating table satisfies the first switching condition for switching the risk level, and the second information of early warning can be determined according to the second risk level and its corresponding second maintenance duration, and a warning can be given with the second information of early warning. The above steps can all refer to step S304 and subsequent contents in Example 2 and will not be repeated here.

The beneficial effect of Embodiment 3 is that, since different first information of early warning is determined for the first risk level according to different first maintenance durations, the first information of early warning reflects the change of the actual risk level situation of the operating table at the first risk level as the first maintenance duration is prolonged. In other words, the first information of early warning that changes with the first maintenance duration in embodiment 3 is closer to the actual risk level situation of the operating table.

The embodiment of this disclosure also provides a system for an early warning of a state of an operating table, as shown in FIG. 5, which includes an operating table 501 and a terminal 502.

The terminal 502 and the operating table 501 are in communicative connection.

The terminal 502 is configured to execute a pre-stored computer program, so as to specifically implement the method for an early warning of a state of an operating table provided in any embodiment of this disclosure.

Optionally, the terminal 502 is a remote control, a wired control for an operating table, a control panel for an operating table, a user terminal, a central station, a monitor, and a control terminal which is used to control an operating table, or a control of another operating table.

The user terminal may be any general electronic device, including but not limited to a mobile phone, a computer, a tablet computer, etc.

The embodiment of this disclosure also provides an operating table, including a main body, and a memory and a controller which are installed at the main body;
wherein the memory is configured to store a computer program, the controller is configured to execute the computer program, so as to specifically implement the method for an early warning of a state of an operating table provided in any one embodiment of this disclosure.

An embodiment of this disclosure also provides a computer-readable storage medium, which includes a stored computer program, wherein when the computer program is executed by a processor, a device, in which the storage medium is arranged, is controlled to execute the method for an early warning of a state of an operating table provided in any embodiment of this disclosure.

The above specific examples are used to illustrate this disclosure, which are only used to help understand this disclosure and are not intended to limit this disclosure. For those skilled in the art, the above specific implementation methods can be modified according to the concept of this disclosure.

## Claims

1. A method for an early warning of a state of an operating table, **characterized in that**, comprising:
acquiring operation-associated data which corresponds to the operating table, wherein the operation-associated data comprises at least one of: basic situation data of a patient, a type of an operation which is to be performed on the operating table, and an estimated duration of the operation;
acquiring an adjustment parameter of a target module of the operating table, wherein the target module is a table top of the operating table or one adjustable module which is part of the operating table;
determining a first risk level which corresponds to the operating table, according to the operation-associated data, the adjustment parameter, and a parameter range of early warning, which range corresponds to the adjustment parameter; and
prompting with first information of early warning;
wherein the first information of early warning corresponds to the first risk level, the first risk level is one of multiple preset risk levels; different risk levels correspond to different information of early warning; different parameter ranges of early warning correspond to different risk levels.

2. The method according to claim 1, **characterized in that**, acquiring operation-associated data which corresponds to the operating table, comprises:
importing, from a data system, the operation-associated data which corresponds to the operating table;
determining, according to an instruction for acquiring data, the operation-associated data which corresponds to the operating table.

3. The method according to claim 1 or claim 2, **characterized in that**, determining a first risk level which corresponds to the operating table, according to the operation-associated data, the adjustment parameter, and a parameter range of early warning, which range corresponds to the adjustment parameter, comprises:
acquiring a target parameter range of early warning, within which range the adjustment parameter of the target module falls; and
determining the first risk level which corresponds to the operating table, according to a risk level which corresponds to the target parameter range of early warning, and according to whether the operation-associated data satisfies a first condition.

4. The method according to claim 3, **characterized in that**, determining the first risk level which corresponds to the operating table, according to a risk level which corresponds to the target parameter range of early warning, and according to whether the operation-associated data satisfies a first condition, comprises:
determining as the first risk level which corresponds to the operating table, a risk level which is higher than the risk level which corresponds to the target parameter range of early warning, when the operation-associated data satisfies the first condition; determining as the first risk level which corresponds to the operating table, the risk level which corresponds to the target parameter range of early warning, when the operation-associated data does not satisfy the first condition; or
determining as the first risk level which corresponds to the operating table, the risk level which corresponds to the target parameter range of early warning, when the operation-associated data satisfies the first condition; determining as the first risk level which corresponds to the operating table, a lowest risk level or a risk level which is lower than the risk level which corresponds to the target parameter range of early warning, when the operation-associated data does not satisfy the first condition;
wherein the operation-associated data satisfying the first condition comprises at least one of: the estimated duration of the operation exceeds a first duration threshold; the basic situation data exceeds preset situation data; the type of the operation is a preset type of operation.

5. The method according to any one of claims 1-4, **characterized in that**, determining a first risk level which corresponds to the operating table, according to the operation-associated data, the adjustment parameter, and a parameter range of early warning, which range corresponds to the adjustment parameter, comprises:
acquiring at least one parameter range of early warning, which range matches with the operation-associated data and corresponds to the adjustment parameter of the target module;
determining, from the at least one parameter range of early warning, a target parameter range of early warning, within which range the adjustment parameter falls; and
determining the first risk level which corresponds to the operating table, according to a risk level which corresponds to the target parameter range of early warning.

6. The method according to any one of claims 1-5, **characterized in that**, acquiring an adjustment parameter of a target module of the operating table, comprises:
acquiring, through detection by a sensor of the operating table, the adjustment parameter of the target module of the operating table; or
determining, according to a control instruction for the operating table, the adjustment parameter of the target module of the operating table.

7. The method according to any one of claims 1-6, **characterized in that**, the adjustment parameter comprises an inclination angle, a movement distance or a folding angle;
the parameter range of early warning comprises an early warning range of inclination angle, an early warning range of movement distance, or an early warning range of folding angle.

8. The method according to claim 7, **characterized in that**,
if the target module is the table top of the operating table, the adjustment parameter is an inclination angle of the table top and/or a movement distance of the table top; a type of the inclination angle of the table top is one of: a head inclination angle, a foot inclination angle, a leftward inclination angle and a rightward inclination angle, of the table top; a type of the movement distance of the table top is one of: an upward movement distance, a downward movement distance, a leftward movement distance and a rightward movement distance, of the table top;
if the target module is a back board of the operating table, the adjustment parameter is a folding angle of the back board;
if the target module is a leg board of the operating table, the adjustment parameter is a folding angle of the leg board;
if the target module is a waist bridge of the operating table, the adjustment parameter is a movement distance of the waist bridge, and a type of the movement distance of the waist bridge is an upward movement distance or a downward movement distance of the waist bridge.

9. The method according to claim 8, **characterized in that**, different types of inclination angle correspond to different parameter ranges of early warning; and/or movement distances of different modules correspond to different parameter ranges of early warning; and/or different types of movement distance correspond to different parameter ranges of early warning; and/or folding angles of different modules correspond to different parameter ranges of early warning.

10. The method according to any one of claims 1-9, **characterized in that**, before prompting with first information of early warning, the method further comprises:
acquiring a first maintenance duration in which the operating table is maintained at the first risk level, and outputting the first maintenance duration.

11. The method according to any one of claims 1-10, **characterized in that**, prompting with first information of early warning, comprises:
acquiring a first maintenance duration in which the operating table is maintained at the first risk level, and/or current situation data of the patient;
determining the first information of early warning, according to the first risk level, and according to the first maintenance duration and/or the current situation data of the patient; and
prompting with the first information of early warning;
wherein, different ranges of maintenance duration correspond to different information of early warning.

12. The method according to claim 10, **characterized in that**, further comprising:
switching the first risk level to a second risk level when a preset switching condition is satisfied, and prompting with second information of early warning;
wherein, the second information of early warning corresponds to the second risk level;
the switching condition comprises at least one of: the first maintenance duration reaches a preset duration threshold; a risk level, which corresponds to a parameter range of early warning of the adjustment parameter after adjusting the target module in the operating table, is the second risk level; the current situation data of the patient is preset situation data.

13. The method according to claim 12, **characterized in that**, prompting with second information of early warning, comprises:
determining the second information of early warning, according to the first risk level and the first maintenance duration, as well as according to the second risk level and a second maintenance duration in which the operating table is maintained at the second risk level; and prompting with the second information of early warning; or
determining the second information of early warning, according to the first risk level and the first maintenance duration, as well as according to the second risk level, a second maintenance duration in which the operating table is maintained at the second risk level, and the current situation data of the patient; and prompting with the second information of early warning; or
determining the second information of early warning, according to the second risk level, as well as according to a second maintenance duration in which the operating table is maintained at the second risk level, and/or the current situation data of the patient; and prompting with the second information of early warning.

14. The method according to claim 12, **characterized in that**, the duration threshold is related to the operation-associated data which corresponds to the operating table; wherein the operation-associated data comprises at least one of: basic situation data of a patient, a type of an operation which is to be performed on the operating table, and an estimated duration of the operation.

15. The method according to any one of claims 1-14, **characterized in that**, determining a first risk level which corresponds to the operating table, according to the operation-associated data, the adjustment parameter, and a parameter range of early warning, which range corresponds to the adjustment parameter; and prompting with first information of early warning; comprise:
when the type of the operation is a preset operation type, determining the first risk level which corresponds to the operating table, according to the operation-associated data, the adjustment parameter, and the parameter range of early warning, which range corresponds to the adjustment parameter.

16. The method according to claim 12, **characterized in that**, the first risk level and the second risk level are different risk levels among high risk level, medium risk level and low risk level.

17. The method according to any one of claims 1-16, **characterized in that**, the information of early warning comprises one or more of: a current risk level, a duration in which the operating table is maintained at the current risk level, a dangerous consequence of the current risk level, and a suggestion for an intervention measure which corresponds to the current risk level;
the suggestion for an intervention measure comprises a suggestion for adjusting the operating table, and/or a suggestion for accelerating the operation.

18. The method according to any one of claims 1-16, **characterized in that**, the information of early warning consists of one or more of: indication voice, indication text, indication icon, indication light and alarm sound.

19. A method for an early warning of a state of an operating table, **characterized in that**, comprising:
acquiring a first risk level which corresponds to the operating table;
acquiring a first maintenance duration in which the operating table is maintained at the first risk level, and/or current situation data of a patient;
determining first information of early warning, according to the first risk level and according to the first maintenance duration and/or the current situation data of the patient; and
prompting with the first information of early warning;
wherein the first information of early warning corresponds to the first risk level, the first risk level is one of multiple preset risk levels; different ranges of maintenance duration correspond to different risk levels; and different risk levels correspond to different information of early warning.

20. The method according to claim 19, **characterized in that**, further comprising:
switching the first risk level to a second risk level when a preset switching condition is satisfied, and prompting with second information of early warning;
wherein, the second information of early warning corresponds to the second risk level;
the switching condition comprises at least one of: the first maintenance duration reaches a preset duration threshold; a risk level, which corresponds to a parameter range of early warning of an adjustment parameter after adjusting a target module in the operating table, is the second risk level; the current situation data of the patient is preset situation data.

21. The method according to claim 20, **characterized in that**, prompting with second information of early warning, comprises:
determining the second information of early warning, according to the first risk level and the first maintenance duration, as well as according to the second risk level and a second maintenance duration in which the operating table is maintained at the second risk level; and prompting with the second information of early warning; or
determining the second information of early warning, according to the first risk level and the first maintenance duration, as well as according to the second risk level, a second maintenance duration in which the operating table is maintained at the second risk level, and the current situation data of the patient; and prompting with the second information of early warning; or
determining the second information of early warning, according to the second risk level, as well as according to a second maintenance duration in which the operating table is maintained at the second risk level, and/or the current situation data of the patient; and prompting with the second information of early warning.

22. The method according to claim 20, **characterized in that**, the duration threshold is related to operation-associated data which corresponds to the operating table; wherein the operation-associated data comprises at least one of: basic situation data of the patient, a type of an operation which is to be performed on the operating table, and an estimated duration of the operation.

23. The method according to any one of claims 19-22, **characterized in that**, the first risk level is determined according to at least one of: operation-associated data which corresponds to the operating table, an adjustment parameter of a target module of the operating table, and a parameter range of early warning, which range corresponds to the adjustment parameter;
wherein the operation-associated data comprises at least one of: basic situation data of the patient, a type of an operation which is to be performed on the operating table, and an estimated duration of the operation.

24. The method according to claim 20, **characterized in that**, the first risk level and the second risk level are different risk levels among high risk level, medium risk level and low risk level.

25. The method according to any one of claims 19-24, **characterized in that**, the information of early warning comprises one or more of: a current risk level, a duration in which the operating table is maintained at the current risk level, a dangerous consequence of the current risk level, and a suggestion for an intervention measure which corresponds to the current risk level;
the suggestion for an intervention measure comprises a suggestion for adjusting the operating table, and/or a suggestion for accelerating the operation.

26. The method according to any one of claims 19-24, **characterized in that**, the information of early warning consists of one or more of: indication voice, indication text, indication icon, indication light and alarm sound.

27. A system for an early warning of a state of an operating table, **characterized in that**, comprising the operating table and a terminal;
wherein, the terminal is in communicative connection with the operating table;
the terminal is configured to execute a pre-stored computer program, so as to specifically implement the method for an early warning of a state of an operating table according to any one of claims 1-26.

28. The system according to claim 27, **characterized in that**, the terminal is a remote control, a wired control of the operating table, a control panel of the operating table, a user terminal, a central station, an anesthesia machine, a monitor, a control terminal, or a control of another operating table.

29. An operating table, **characterized in that**, comprising a main body, and a memory and a controller which are installed at the main body;
wherein the memory is configured to store a computer program, the controller is configured to execute the computer program, so as to specifically implement the method for an early warning of a state of an operating table according to any one of claims 1-26.

30. A computer-readable storage medium, **characterized in that**, comprising a stored computer program, wherein when the computer program is executed by a processor, a device in which the storage medium is arranged, is controlled to execute the method for an early warning of a state of an operating table according to any one of claims 1-26.
